Europäisches Patentamt

European Patent Office

Office européen des brevets

(19)

(11) Numéro de publication : **0 384 090 B1**

# FASCICULE DE BREVET EUROPEEN

(12)

(45) Date de publication du fascicule du brevet :
**16.06.93 Bulletin 93/24**

(51) Int. Cl.⁵ : **C12M 1/40,** A61B 5/00

(21) Numéro de dépôt : **89403291.1**

(22) Date de dépôt : **28.11.89**

(54) **Biocapteur à enzyme destiné à la mesure directe d'au moins un paramètre biochimique de la peau.**

(30) Priorité : **16.12.88 FR 8816664**

(43) Date de publication de la demande :
**29.08.90 Bulletin 90/35**

(45) Mention de la délivrance du brevet :
**16.06.93 Bulletin 93/24**

(84) Etats contractants désignés :
**AT BE CH DE ES GB GR IT LI NL SE**

(56) Documents cités :
**EP-A- 0 154 664
EP-A- 0 248 680
US-A- 4 020 830
US-A- 4 071 020**

(73) Titulaire : **L'OREAL
14, Rue Royale
F-75008 Paris (FR)**

(72) Inventeur : **Bernard, Dominique
4, rue de l'Epargne
F-60200 Compiègne (FR)**
Inventeur : **Kermici, Michel
36, rue de Picpus
F-75012 Paris (FR)**
Inventeur : **Pruniéras, Michel
72, avenue Mozart
F-75016 Paris (FR)**

(74) Mandataire : **Peuscet, Jacques et al
Cabinet Peuscet 68, rue d'Hauteville
F-75010 Paris (FR)**

EP 0 384 090 B1

## Description

La présente invention porte sur un biocapteur à enzyme destiné à la mesure directe des paramètres biochimiques de la peau, ainsi que sur le procédé de mesure correspondant et le procédé de préparation de la membrane enzymatique nécessaire.

On connaît un grand nombre de techniques permettant de mesurer in vivo, de façon non invasive, des paramètres physiques de la peau. Si beaucoup de données sont disponibles sur ces paramètres physiques, peu le sont en revanche sur les signaux biologiques directement accessibles sur le stratum corneum, et les méthodes de dosage permettant l'acquisition directe de tels signaux sont pratiquement inexistantes à l'heure actuelle.

Ces facteurs biochimiques présentent cependant un intérêt de diagnostic. Ainsi, par exemple, le métabolisme énergétique de la peau comporte un cycle anaérobie prépondérant avec formation d'acide lactique à partir d'acide pyruvique ; cet acide lactique se retrouve dans les couches suprabasales de l'épiderme, ainsi qu'au niveau du stratum corneum. L'un des rôles décrits pour le lactate exogène est celui d'agent naturel d'hydratation de la peau en raison de ses propriétés hygroscopiques (Van Scott et al, J. Am. Acad. Dermatol. 1984, 11. 869 ; et Takahashi et al, J. Soc. Cosmet. Chem. 1985, 36, 177). On sait, par ailleurs, qu'une augmentation du taux de lactate est un signe d'hyperprolifération cellulaire dans les couches profondes.

L'intérêt d'un dispositif réalisant, dans les meilleures conditions, une mesure du lactate sur la peau serait donc, notamment, de pouvoir juger de l'importance de ce métabolite sur l'hydratation de la peau et de son possible rôle diagnostic ou curatif. Les meilleures conditions, dont il est question ci-dessus, sont celles d'une mesure directe, sans étape de prélèvement et sans apport de réactif sur la peau. De plus, il peut s'avérer intéressant de pouvoir réaliser des mesures répétées sur un même site, par exemple, à une minute d'intervalle, afin d'observer des phénomènes d'épuisement ou de diffusion facilitée du lactate cutané.

En réalité, il serait utile, d'une manière générale de disposer de moyens de mesure directe sur le revêtement cutané de toutes les molécules d'intérêt dermatologique ou cosmétique, comme, par exemple, l'urée, le cholestérol ou les acides aminés. Les études à mener peuvent aussi, également de façon générale, concerner la rémanence, l'absorption et le relargage d'un des composés en cause.

Or, si l'on reprend l'exemple du lactate, les méthodes de détermination habituelles de cette substance, en dehors du fait qu'elles imposent une étape de prélèvement, sont sensibles mais relativement lentes (Barker et al, J. Biol. Chem. 1941, 138, 535). Les mêmes désavantages peuvent être attribués à la méthode enzymatique utilisant la lactate deshydrogénase, ci-après dénommée LDH, (E.C. 1.1.1.27), qui est basée sur la détection à 340 nm du coenzyme, le nicotinamide - adénine - dinucléotide réduit ; de plus, des interférences liées à la présence d'effecteurs de la LDH dans l'échantillon ou à la turbidité de cet échantillon peuvent nuire à la sensibilité de la mesure (Gutman et al, dans H.U. Bergmeyer (Ed.), Methods of Enzymatique Analysis, Verlag Chemie, Weinham, 2ème édition 1974, 264).

Par ailleurs, les capteurs électrochimiques, associés à un film, dans lequel est immobilisée une enzyme, sont déjà largement utilisés pour doser des composés se trouvant dans les milieux biologiques. Ainsi, il existe, à l'heure actuelle, un nombre important d'électrodes enzymatiques spécifiques du L-lactate, trouvant une application dans le domaine des industries agro-alimentaires, ainsi que dans celui des analyses médicales. Des électrodes à enzyme utilisant l'un des quatre systèmes enzymatiques envisageables pour de telles applications ont été décrites :

- la LDH [Durliatt et al, Anal. Chem. 1980, 52, 2109] ;
- le cytochrome b2 (E.C. 1.1.2.3) [Shinbo et al, Anal. Chem. 1979, 51, 100] ;
- la lactate 2-mono-oxygénase (E.C.1.13.12.4) de Mycobacterium smegmatis qui catalyse la réaction suivante :

$$L\text{-lactate} + O_2 \rightarrow \text{acétate} + CO_2 + H_2O$$

associée à une électrode de Clark [Mascini et al, Anal. Chem. Acta 1984, 157, 45] ;
- la lactate oxydase (E.C.1.1.3.2) de Pediococcus sp., avec laquelle on obtient les meilleurs résultats du point de vue de la stabilité, de la sensibilité et de la fiabilité et qui catalyse la réaction suivante :

$$L\text{-lactate} + O_2 \rightarrow \text{pyruvate} + H_2O_2$$

associée à une détection de la pression partielle en oxygène [Mizutani et al, Anal. Chem. 1983, 55, 35] ou à une détection de $H_2O_2$ [Mullen et al, Clin. Chem. Acta 1986, 157, 191].

Pour atteindre l'objectif indiqué ci-dessus, qui est celui de pouvoir déterminer, par des mesures directes, les paramètres biochimiques de la peau, il était intéressant d'utiliser la méthode connue suivant laquelle l'échantillon à doser est amené au contact d'une membrane, dans laquelle est immobilisée (comme décrit par Romette, Thèse de doctorat, Université de Compiègne, 1986) une enzyme, qui catalysera la transformation de la substance à doser avec, dans le cas notamment du L-lactate, consommation d'oxygène et production d'$H_2O_2$, des moyens étant prévus pour détecter un phénomène provoqué par la consommation d'oxygène ou

la production d'H$_2$O$_2$, par exemple des moyens de détection ampérométriques de la diminution de la pression partielle d'oxygène, un exemple en étant l'électrode de Clark, décrite plus en détail ci-après, la membrane enzymatique étant appliquée à l'extrémité sensible de l'électrode.

En effet, la sensibilité de tels biocapteurs est le plus souvent importante. Toutefois, l'application directe de l'électrode sur la surface cutanée s'est révélée impossible, par suite de difficultés venant de la compression du film d'électrolyte, de la résistance mécanique de la membrane et de la mise en solution du substrat.

La Société déposante a pallié ces inconvénients en associant à un biocapteur à enzyme du type précité, une cellule de mise en solution du substrat à doser, dans laquelle on peut faire circuler un milieu liquide approprié, tel qu'un tampon, cette cellule comportant une ouverture qui, en position de mesure, est obturée par une zone de revêtement cutané, laquelle constitue le site de mesure.

Il découle également de la mise en place de cette cellule à circulation de fluide, fermée par une zone de peau au moment de la mesure, que l'injection du tampon de mesure peut être rendue automatique, et surtout que l'on peut effectuer plusieurs cycles de mesure sur un même site, ce qui répond à l'un des objectifs importants indiqués ci-dessus.

La présente invention a donc d'abord pour objet un biocapteur à enzyme destiné à la mesure directe d'au moins un paramètre biochimique de la peau ledit biocapteur comprenant, d'une part, une membrane, dans laquelle est immobilisée une enzyme destinée à catalyser la transformation d'un substrat intervenant dans le métabolisme cutané, et, d'autre part, des moyens pour détecter et mesurer un phénomène provoqué par ladite transformation, qui est représentatif de la concentration du substrat à doser, celui-ci étant amené au contact de la membrane enzymatique, caractérisé par le fait qu'il comprend une cellule de mesure comportant une ouverture, cellule dont une zone de paroi est constituée par ladite membrane enzymatique et dont l'ouverture susmentionnée est apte à être obturée, lors de la mesure, par une zone de revêtement cutané, ladite cellule étant associée à des moyens pour y faire circuler un liquide de mise en solution du substrat à doser.

Dans un tel système de mesure directe, le résultat obtenu est le reflet, d'une part, de la concentration du substrat cutané, que l'on veut doser, et d'autre part, des contraintes diffusionnelles, que la peau peut opposer à la diffusion de ce substrat vers la cellule de mesure, ces contraintes diffusionnelles pouvant être reliées au type de peau.

Dans un mode de réalisation préféré, suivant lequel la transformation du substrat à doser est une réaction qui consomme de l'oxygène et/ou produit de l'H$_2$O$_2$, les moyens de détection et de mesure sont constitués par une électrode mesurant la diminution de la pression partielle d'oxygène ou l'augmentation de la concentration en H$_2$O$_2$, la membrane enzymatique étant appliquée contre la partie sensible d'extrémité de ladite électrode, le liquide de mise en solution étant un tampon susceptible de fournir de l'oxygène ; la cellule de mesure est avantageusement constituée par une bague, à une extrémité de laquelle est montée, de manière étanche, la partie sensible d'extrémité de ladite électrode, l'autre extrémité de ladite bague constituant l'ouverture de la cellule.

Dans le biocapteur de l'invention, la membrane enzymatique peut être avantageusement constituée d'un film-support de matériau hydrophobe à perméabilité sélective vis-à-vis des gaz et d'un film porteur de l'enzyme formé par réticulation d'au moins une protéine inerte par un agent pontant, comme le glutaraldéhyde ; le film-support de la membrane enzymatique est disposé du côté de l'électrode et le film porteur de l'enzyme est disposé en vis-à-vis de l'ouverture de la cellule de mesure.

On peut mentionner, en particulier, un biocapteur selon l'invention destiné au dosage du L-lactate cutané, l'enzyme étant la L-lactate oxydase, notamment la L-lactate oxydase de Pediococcus sp., et l'électrode étant une électrode mesurant la diminution de la pression partielle d'oxygène (électrode dite de Clark) ; le film porteur de l'enzyme contient de 0,15 à 0,50 UI de L-lactate oxydase par cm$^2$ de membrane. L'épaisseur du film porteur est, de préférence, comprise entre 30 et 50 μm ; la protéine inerte est, par exemple, la gélatine.

Dans le cas particulier indiqué, le matériau hydrophobe constitutif du film-support est, de préférence, le polypropylène ou le polytétrafluoréthylène. L'épaisseur du film-support est avantageusement comprise entre 6 et 15 μm.

Pour la préparation d'une membrane enzymatique destinée à un biocapteur selon l'invention prévu pour le dosage du L-lactate, on procède de la manière suivante :

a) on prépare une solution aqueuse renfermant, par ml,

de 4 à 20 UI de L-lactate oxydase et

de 30 à 70 mg de gélatine ;

b) on étale ladite solution sur un film-support à raison de 10 à 50 μl/cm$^2$ et on fait sécher ce revêtement ;

c) sur le revêtement sec obtenu, on verse une solution aqueuse de glutaraldéhyde ayant une concentration comprise entre 0,5 et 2 % en poids et on laisse réticuler pendant un temps compris entre 1,5 et 4 minutes.

La présente invention a également pour objet un procédé pour la mesure directe d'au moins un paramètre biochimique de la peau à l'aide d'un biocapteur tel que défini ci-dessus, caractérisé par le fait qu'on applique ledit biocapteur sur une zone choisie du revêtement cutané, de façon que l'ouverture de la cellule de mesure soit fermée par ladite zone de revêtement cutané ; on injecte dans ladite cellule un liquide apte à solubiliser le substrat cutané que l'on veut doser et dont la composition favorise la catalyse ; après remplissage de la cellule de mesure, on effectue la détection et la mesure du phénomène provoqué par la transformation du substrat due à la catalyse de l'enzyme de la membrane ; puis on fait circuler le liquide de solubilisation du substrat.

On peut avantageusement répéter le cycle de mesure précité sur le même site, auquel cas on n'expose pas la membrane enzymatique à l'air entre deux cycles successifs.

Dans le cas où le biocapteur est destiné à mesurer le lactate cutané en mettant en oeuvre la lactate-oxydase, on peut avantageusement prévoir que l'on utilise, comme liquide de mise en solution du lactate cutané, un tampon ayant un pH compris entre 6 et 8 ; que l'on enregistre la variation de pression partielle d'oxygène $pO_2$ en fonction du temps pendant la période de non-circulation du tampon dans la cellule ; qu'on définit sur l'enregistrement le point A ayant pour abscisse le début d'injection du tampon et pour ordonnée la valeur maximum de $pO_2$ ; qu'on définit la droite D liant A au minimum B de la courbe enregistrée et qu'on mesure sa pente P pour l'utiliser comme valeur représentative du taux de lactate cutané. Dans ce type de mesure, la circulation du tampon est, de préférence, arrêtée pendant un temps compris entre 3 et 6 minutes.

Il convient de préciser que le biocapteur selon l'invention peut être avantageusement utilisé notamment pour la mesure d'un paramètre de l'hydratation cutanée et pour l'estimation de la sécrétion sudorale. Dans le cas où l'on mesure un paramètre de l'hydratation cutanée, le biocapteur peut être particulièrement utile pour aider à diagnostiquer la sécheresse d'une peau. Dans le cas où l'on estime la quantité de sécrétion sudorale, le biocapteur permet d'évaluer l'efficacité des produits inhibiteurs de sudation. Ces utilisations sont basées sur le fait que le lactate se trouve en forte concentration dans la sécrétion sudorale (15 à 40 m/M), la mesure des quantités de lactate permettant donc d'avoir une estimation directe de la sécrétion sudorale.

Pour mieux faire comprendre l'objet de l'invention, on va décrire plus en détail ci-après, à titre purement illustratif et non limitatif, la réalisation d'un biocapteur à usage cutané pour le dosage du L-lactate, en se référant au dessin annexé :

Sur ce dessin :
- les figures 1, 1A et 2 concernent l'électrode utilisée ;
- les figures 3 à 13 représentent différentes courbes obtenues lors des mesures effectuées, entre autres, pour optimiser ladite électrode.

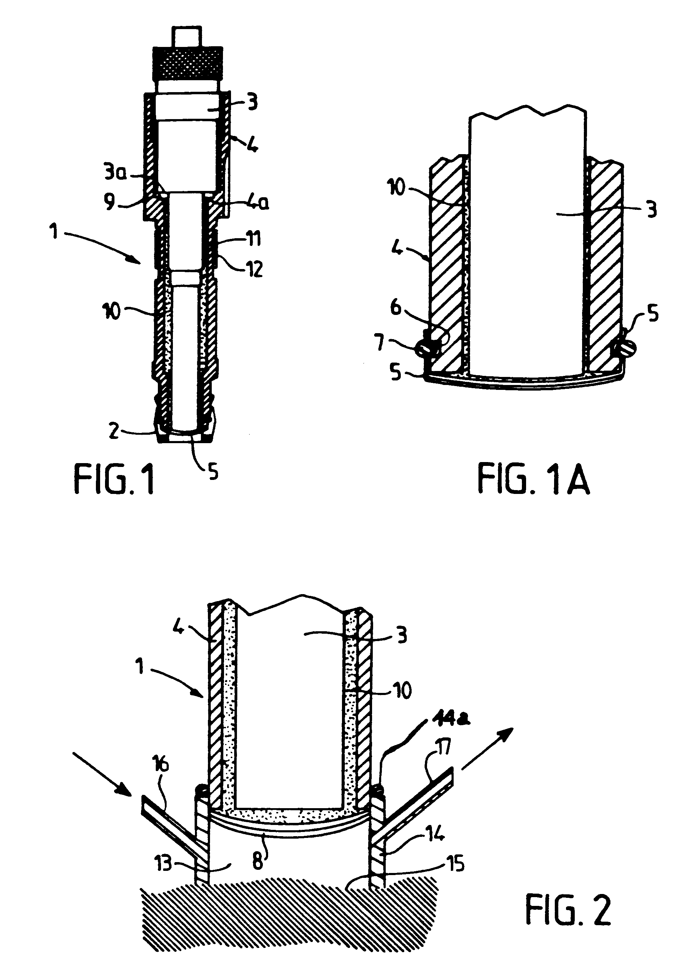

La figure 1 représente une électrode de Clark 1 de type connu, qui convient, comme élément de base, pour la réalisation d'un biocapteur de L-lactate selon l'invention, en vue de la mesure du L-lactate cutané. L'électrode 1 comprend un corps 3 logé dans une enveloppe 4. L'extrémité libre de l'électrode 1 est recouverte par un film hydrophobe 5, qui ne laisse passer que l'oxygène et qui est, par exemple, un film de polypropylène ou de polytétrafluoréthylène ; cette extrémité de l'électrode est protégée par un embout 2 qui permet néanmoins les mesures. La fixation du film 5 est montrée plus en détail sur la figure 1a, laquelle représente, à échelle agrandie, la partie d'extrémité correspondante de l'électrode 1, sans l'embout 2. L'enveloppe 4 de l'électrode 1 comporte, au voisinage de son extrémité, une rainure annulaire 6 destinée à recevoir un joint torique 7, avec interposition du film 5. Au voisinage de son extrémité opposée au film 5, le corps 3 de l'électrode 1 comporte un épaulement annulaire 3a en appui sur une portée correspondante 4a de l'enveloppe 4, avec interposition d'un joint 9 ; entre ledit joint 9, l'enveloppe 4 et le corps 3 de l'électrode 1, on a ménagé un espace rempli d'électrolyte 10, l'électrolyte 10 étant interposé entre l'électrode 1 et le film 5. L'enveloppe 4 comporte, par ailleurs, une ouverture latérale 11, en vue de réaliser la purge de l'électrolyte, et l'équilibre de la pression de l'électrolyte, l'ouverture 11 étant normalement obturée de manière étanche par une bague 12.

Sur la figure 2, est représentée, de façon schématique, l'extrémité de l'électrode de Clark correspondant aux figures 1 et 1a, telle qu'on l'a modifiée pour réaliser un biocapteur selon l'invention. Sur ladite extrémité, on a adapté, conformément à la présente invention, une cellule de mesure 13 à circulation, constituée par un anneau sans fond 14. L'extrémité de l'électrode 1 est disposée, de manière étanche grâce à la présence d'un joint 14a, à une extrémité dudit anneau 14, dont l'autre extrémité est destinée à être mise en appui sur la surface cutanée 15 lors de la mesure. La cellule 13 est destinée à être traversée par un flux de tampon de mesure amené par la tubulure d'entrée 16 et évacué par la tubulure de sortie 17. Ce circuit de fluide comporte des moyens (non représentés) assurant l'injection du tampon de mesure par la tubulure 16, tels qu'une pompe.

Pour réaliser un biocapteur selon l'invention, on fabrique d'abord une membrane enzymatique appropriée puis on en équipe une électrode de Clark telle que ci-dessus décrite. On va donner ci-après un exemple précis pour ces deux étapes.

1) Réalisation de la membrane enzymatique

On prépare une solution à 5 % (poids/volume) de gélatine d'osséine Chaulée 250 blooms (Rousselot, France), dans un tampon phosphate 0,02 M, pH 6,8 (cette solution est conservée au maximum pendant une semaine à 4°C avant son utilisation).

On solubilise, dans son flacon de 100 UI (référence : Sigma, n° L-0638), la L-lactate oxydase de Pediococcus Sp. lyophilisée, par 500 µl de tampon phosphate 0,1 M, pH 7,1 et on la conserve à 4°C avant son utilisation.

On prépare extemporanément une solution de glutaraldéhyde à 1,25 % (poids/volume) dans un tampon phosphate 0,02 M, pH 6,8 à partir de glutaraldéhyde (Sigma n° G-5882) en solution aqueuse à 25 % en poids, de qualité 1.

On chauffe la solution de gélatine ainsi préparée à 45°C pendant 5 minutes. On la refroidit ensuite à 30°C. On prélève alors 1 ml de cette solution et on homogénéise le mélange avec 50 µl de la solution enzymatique (10 UI). On verse le mélange homogénéisé sur un film-support de polypropylène (Bolloré), d'une épaisseur de 6 µm, fixé sur une plaque de verre parfaitement plane. La solution est étalée sur une surface d'exactement 35 cm², délimitée par un ruban adhésif. L'application d'un flux d'air, à température ambiante, pendant 40 minutes, permet de sécher la membrane.

On réalise alors la réticulation de cette membrane par la solution de glutaraldéhyde en appliquant 10 ml de celle-ci sur la membrane pendant exactement 3 minutes. Un rinçage avec 3 litres d'eau distillée est alors effectué par écoulement continu sur la membrane pour stopper la réticulation et éliminer l'excès de l'agent pontant.

On peut alors découper 12 membranes aptes à être appliquées sur l'électrode de Clark utilisée, et les conserver à 4°C, dans un tampon phosphate 0,1 M, pH 7,1 jusqu'à leur ajustement à l'extrémité de l'électrode et à leur utilisation.

### 2) Préparation et mise en oeuvre de l'électrode à enzyme

On utilise une électrode de Clark (Radiometer, Copenhague, Danemark, modèle E 5046) telle que représentée sur la figure 1. On recouvre la zone sensible à l'oxygène de cette électrode avec une membrane enzymatique 8, telle que préparée à l'étape précédente, le film-support 5 de polypropylène, qui est sélectif aux gaz, étant du côté de l'électrode.

Pour la mesure, l'électrode ainsi obtenue est associée à une cellule de mesure thermostatée (Radiometer D616), afin de constituer le biocapteur tel que représenté sur la figure 2 précédemment décrite.

La réaction enzymatique, ayant lieu selon l'invention à l'extrémité de l'électrode de Clark est la suivante :

$$\text{L-lactate} + O_2 \rightarrow \text{pyruvate} + H_2O_2.$$

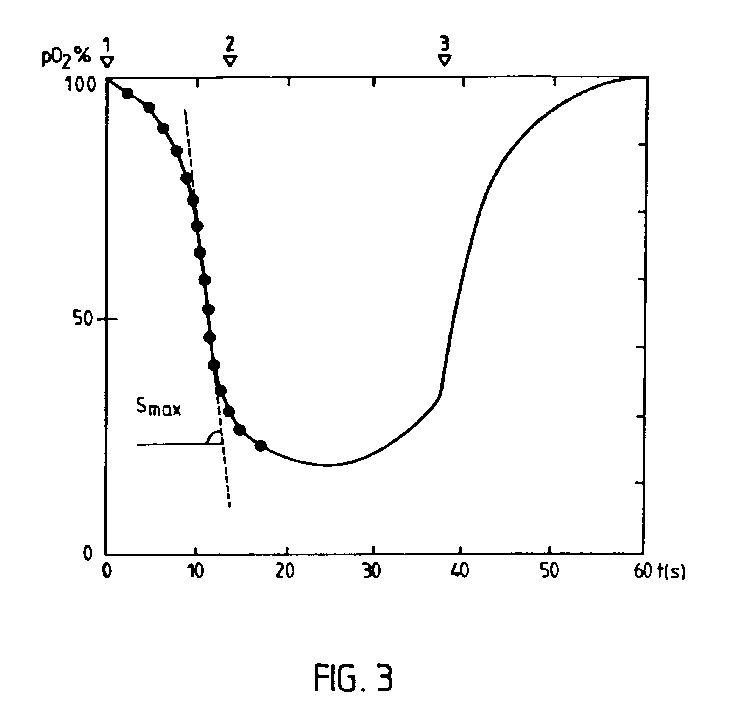

Pour l'étalonnage du biocapteur et son étude, on alimente la cellule 13 par un flux continu d'une solution aqueuse de lactate de concentration connue. La donnée étalonnable, pour ces mesures en flux continu, est la pente Smax au point d'inflexion de la courbe du signal de $pO_2$ (pression partielle d'oxygène) enregistré par l'électrode. La figure 3 représente une courbe type obtenue au cours d'un cycle de mesure en flux continu : en abscisse, on a porté le temps en secondes et en ordonnée, la pression partielle d'oxygène ($pO_2$) exprimée en pourcentage par rapport à la pression $pO_2$ initiale du tampon. Dans ce cycle de mesure, au temps (1), du L-lactate est introduit dans la cellule avec du tampon phosphate 0,1 M, pH 7. Au temps (2), la cellule est rincée avec du tampon. Au temps (3), de l'air est introduit dans la cellule, pour assurer à nouveau la saturation d'oxygène à 100% de la membrane utilisée. Le traitement mathématique du signal est connu (Kernevez et al., Biotech. Bioeng. 1983, 25, 845). Le traitement automatique du signal, le circuit fluidique et l'acquisition des courbes d'étalonnage sont contrôlés par un micro-ordinateur, de façon connue.

Le cycle de mesure en flux continu du biocapteur préparé comme indiqué ci-dessus comprend séquentiellement les trois phases correspondant aux temps (1) à (3) indiqués ci-dessus en référence avec la figure 3, étant entendu qu'il commence par l'étape d'exposition à l'air de la membrane. L'étape d'exposition à l'air est effectuée afin d'augmenter la concentration en oxygène à l'intérieur de la membrane active, laquelle devient alors vingt fois plus importante que dans l'eau pour la même pression partielle d'oxygène (Belgith, H. Thèse de Doctorat, Université de Compiègne, 1985).

Comme on va le voir ci-après, pour des mesures répétitives directes sur une même localisation cutanée, l'étape d'exposition à l'air de la membrane est supprimée pour des raisons pratiques.

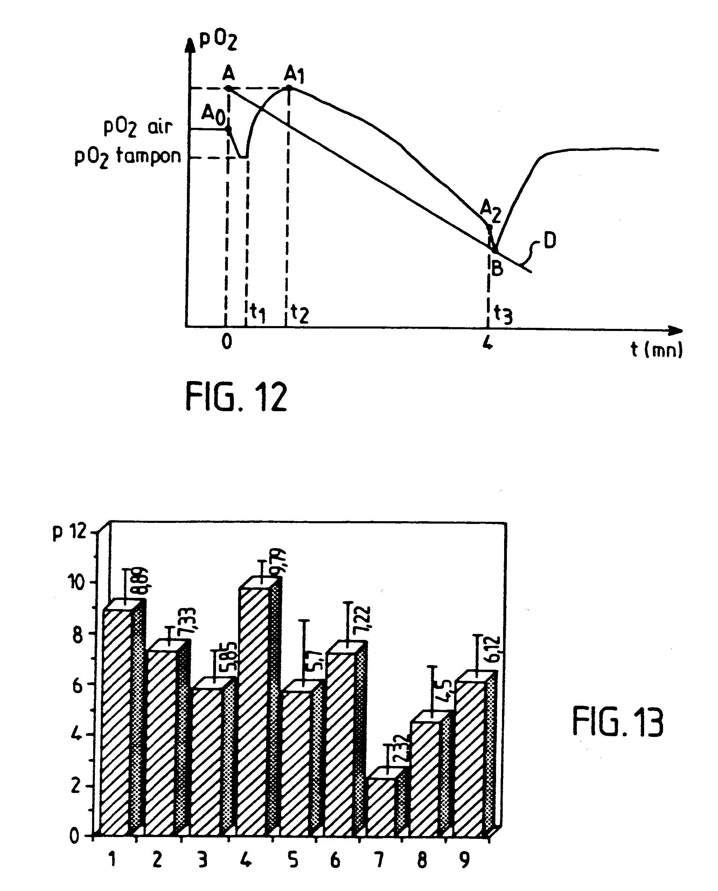

L'utilisation effective du biocapteur selon l'invention est, dans cet exemple, réalisée comme suit. Le biocapteur, dont la cellule de mesure a un volume de 100 µl pour une surface d'application de 0,6 cm2, est appliqué sur la peau. On injecte du tampon phosphate 0,1 M, pH 7,1 à température ambiante (21°C), dans la cellule de mesure, grâce à une pompe péristaltique (Gilson Minipuls 3) assurant un débit de remplissage de l'ordre de 1,2 ml/min, constant et parfaitement reproductible. Une fois la cellule remplie, la pompe est stoppée ; on enregistre en fonction du temps la valeur $pO_2$ (voir courbe de la figure 12). Initialement $pO_2$ a la valeur correspondant à l'air ($pO_2$ air). Au temps 0 on introduit le tampon (point Ao de la courbe) : la valeur de $pO_2$ décroît

pour se stabiliser à la valeur de la pression partielle d'oxygène dans le tampon ($pO_2$ tampon). Puis on arrête la pompe de circulation du tampon au temps $t_1$ : $pO_2$ augmente ce qui traduit l'équilibrage de la température du tampon avec la température cutanée ; mais depuis le temps 0, la membrane enzymatique a commencé son action qui tend à diminuer la valeur $pO_2$ donnée par l'électrode, cette action étant masquée par les phénomènes ci-dessus définis. La valeur $pO_2$ passe donc par un maximum $A_1$ résultant des deux actions antagonistes simultanées, ce maximum étant atteint pour un temps $t_2$. Après quoi, $pO_2$ décroît en raison de l'action enzymatique ; cependant, cette décroissance est freinée par une fourniture d'oxygène du tampon ; à chaque instant, on a donc un équilibre ; mais la concentration en lactate dans la cellule de mesure augmente par diffusion du lactate au cours du temps à partir du revêtement cutané et la courbe obtenue traduit donc une succession d'états stationnaires. Au bout d'un temps suffisant, par exemple environ 4 minutes, on fait recirculer le tampon : $pO_2$ décroît rapidement pendant un court instant, ce qui traduit l'hétérogénéité du milieu dans la cellule de mesure puis, après un minimum B, réaugmente pour revenir approximativement à la valeur de la $pO_2$ du tampon de mesure.

On a constaté que la donnée la plus représentative du taux de lactate cutané est la pente P de la droite D qui passe par les deux points A et B du graphique de la figure 12 :

. point A    : abscisse = temps 0

ordonnée = valeur du maximum $A_1$ au temps $t_2$

. point B    : minimum B atteint par la courbe après recirculation du tampon.

La valeur P peut être utilisée pour exprimer le taux de lactate diffusant en nmoles/mn/cm$^2$ à condition de se rapporter à un étalonnage effectué en mode stationnaire, c'est-à-dire à la valeur plateau de la $pO_2$ obtenue lorsque l'oxygène consommé par la réaction enzymatique est exactement compensé par l'oxygène fourni par le milieu de mesure.

Comme indiqué précédemment, étant donné que chaque mesure est suivie d'une phase de circulation du tampon, on peut effectuer plusieurs cycles de mesure successifs sur un même site.

La figure 13 donne, à titre indicatif, les valeurs obtenues (pentes P) pour des mesures effectuées sur l'avant-bras de neuf individus de sexe féminin. Ces valeurs sont des valeurs cumulées résultant de quatre mesures par individu (2 mesures successives pour une même localisation sur chaque avant-bras).

On va donner ci-après un certain nombre d'indications qui ont conduit à la mise au point du biocapteur optimisé, qui vient d'être décrit, et à son utilisation optimale.

## I. OPTIMISATION DU BIOCAPTEUR

### 1) Optimisation des étapes d'immobilisation de l'enzyme :

La bonne stabilité, mécanique et biochimique, de la membrane enzymatique active est la condition essentielle rendant possible une utilisation analytique du biocapteur. Cette stabilité dépend de certains facteurs physiques, chimiques ou biochimiques :

### a) La concentration en enzyme dans la membrane active :

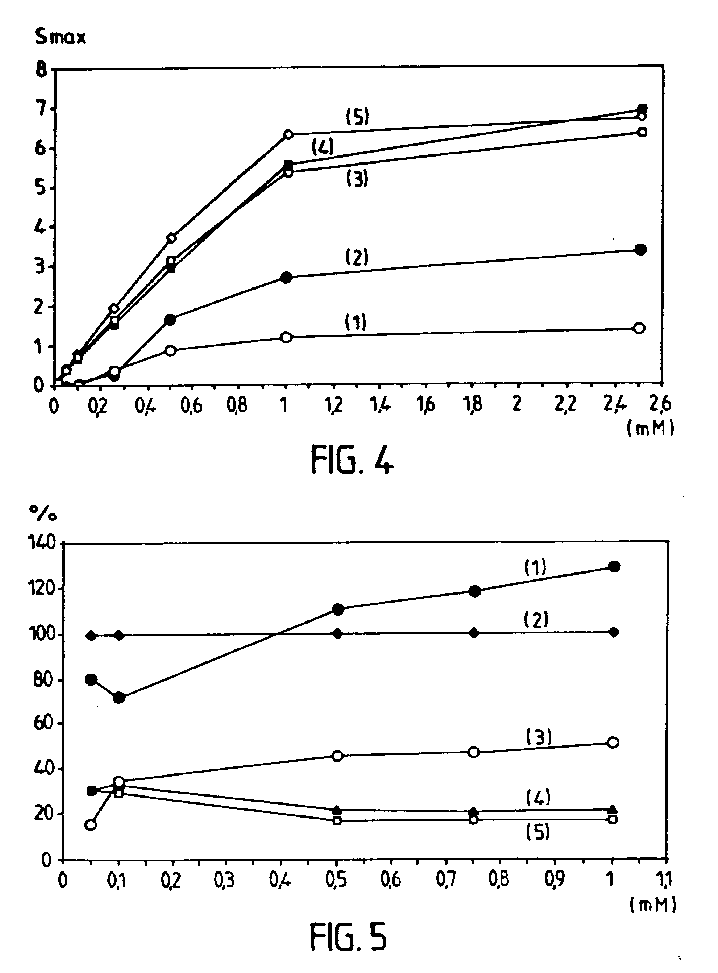

La figure 4 du dessin annexé représente différentes courbes d'étalonnage (1) à (5), qui ont été établies avec des membranes contenant différentes concentrations de L-lactate oxydase et dans les conditions communes suivantes :

. Tampon phosphate : 0,1 M, pH 7,1 ;

. Temps de réticulation à l'aide d'une solution de glutaraldéhyde à 1,25 % (poids/volume) : 3 minutes ;

. Concentration de la solution de gélatine : 5 % (poids/volume) ;

. Température : 21°C ;

### Légende de la figure 4 :

. Abscisse : concentration de L-lactate en mM ;

. Ordonnée : pente Smax définie sur la courbe de la figure 3 ;

. Quantité de L-lactate oxydase (exprimée en UI pour 35 cm$^2$ de film de gélatine) pour les courbes (1) à (5) : respectivement 1 ; 4 ; 10 ; 20 et 30.

Les résultats montrent que des concentrations en enzyme élevées (> 4 UI/35 cm2) peuvent être utilisées pour mesurer des concentrations en lactate relativement basses (zone de linéarité de la réponse située entre $1 \times 10^{-5}$ et $1 \times 10^{-3}$ M). Quand la concentration en lactate varie entre 1 et 3 mM, des membranes contenant 4 UI doivent être utilisées.

Par ailleurs, avec un excès d'enzyme (> 4 UI), la réponse de l'électrode devient indépendante de la quantité d'enzyme. Ceci est lié à deux facteurs :
- la saturation de la membrane protéique en sites actifs ; et
- l'augmentation de la masse protéique enzymatique et donc de l'épaisseur de la membrane (une augmentation de l'activité enzymatique dans la membrane ne pouvant être obtenue à masse protéique enzymatique constante).

Le facteur limitant de la réponse de l'électrode devient alors le coefficient de diffusion du substrat. De telles conditions expérimentales (saturation en enzyme) peuvent être utilisées de façon avantageuse, permettant d'obtenir des électrodes à réponse plus stable, une petite perte d'activité enzymatique ou un relargage partiel de l'enzyme n'ayant que peu d'effet sur le signal.

Compte tenu de ces résultats, dans les études suivantes, il a été choisi, pour chaque paramètre étudié, une concentration en enzyme de 10 UI pour 35 cm$^2$.

b) Le temps de réticulation avec le glutaraldéhyde :

Cette étape de la préparation de la membrane enzymatique est très importante, car le glutaraldéhyde, qui peut bloquer certains groupements amines libres d'amino-acides impliqués dans le site actif, est appliqué sur le film protéique pendant un laps de temps fixé.

La figure 5 du dessin annexé représente différentes courbes de réponse (1) à (5) du biocapteur à L-lactate oxydase pour différents temps de réticulation à l'aide d'une solution aqueuse de glutaraldéhyde à 1,25 % (poids/volume) (100 % de la réponse pour un temps de réticulation de 3 minutes), dans les conditions communes suivantes :
. Tampon phosphate : 0,1M, pH 7,1 ;
. Concentration de la solution de gélatine : 5 % (en poids/volume) ;
. Température : 21°C.

Légende de la figure 5 :

. Abscisse : concentration de L-lactate (en mM) ;
. Ordonnée : pourcentage de la réponse par rapport à celle obtenue pour un temps de réticulation de 3 minutes ;
. Temps de réticulation pour les courbes (1) à (5) respectivement 1,5 ; 3 ; 5 ; 7,5 ; et 10 mn.

Cette figure montre que les réponses les plus importantes du biocapteur ont été obtenues pour des temps de réticulation de 1,5 à 3 minutes. Des temps de réticulation plus importants impliquent une dénaturation plus poussée de l'enzyme et une cohésion plus importante de la membrane, limitant le taux de diffusion du lactate. Ces deux facteurs conduisent à une décroissance du signal, qui est proportionnelle au temps de réticulation pour des temps de contact supérieurs à 3 minutes. La mise en évidence de meilleures propriétés mécaniques pour un temps de réticulation de 3 minutes par rapport à 1,5 minute a conduit à choisir celui-ci pour la réalisation des membranes dans l'exemple de réalisation précédemment décrit.

c) La concentration en gélatine :

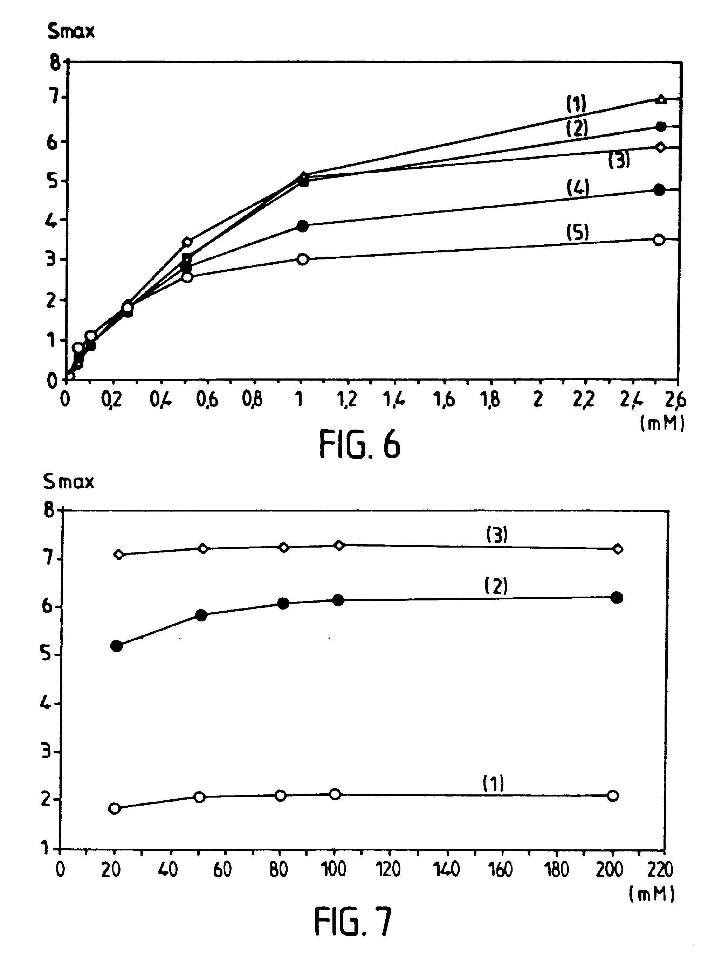

On a testé des membranes contenant la même quantité d'enzyme (10 UI pour 35 cm$^2$) avec des quantités variables de gélatine (solutions de 3 à 7 % pour la fabrication des membranes selon le processus indiqué dans l'exemple précédemment décrit). La figure 6 représente des courbes d'étalonnage dans le cas d'utilisation de solutions de gélatine à différents pourcentages [ courbes (1) à (5)], dans les conditions communes suivantes :
. Tampon phosphate : 0,1 M, pH 7,1 ;
. Temps de réticulation à l'aide d'une solution de glutaraldéhyde à 1,25 % (poids/volume) : 3 minutes ;
. Température : 21 °C.

Légende de la figure 6 :

. Abscisse : concentration de L-lactate en mM ;
. Ordonnée : pente Smax (définie sur la figure 3) ;
. Pourcentage de gélatine dans les solutions de gélatines utilisées pour les courbes (1) à (5), respectivement 3 ; 4 ; 5 ; 6 et 7.

Différents facteurs doivent être considérés :
- premièrement, la densité finale en protéine enzymatique décroît avec l'augmentation du pourcentage de gélatine ; donc, théoriquement le film protéique doit être plus actif dans des membranes les plus fines ;

- deuxièmement, l'épaisseur de la membrane augmente quand la concentration en gélatine augmente, le coefficient de diffusion du lactate et, par conséquent, la réponse de l'électrode sont modifiés ;
- troisièmement, l'effet protecteur contre les propriétés inactivatrices du glutaraldéhyde (temps de réticulation égal à 3 minutes) augmente parallèlement au pourcentage de gélatine, l'activité catalytique est donc mieux préservée dans des films plus épais.

Un compromis entre ces différents facteurs doit donc être trouvé. La figure 6 montre que la meilleure réponse de l'électrode est obtenue, pour des concentrations en substrat supérieures à 0,2 mM, avec des membranes à 5 % en gélatine.

Les recommandations générales pour la réalisation d'électrodes à enzyme selon l'invention sont d'utiliser une enzyme d'activité spécifique aussi élevée que possible pour assurer des cinétiques rapides dans une membrane aussi fine que réalisable pour assurer les meilleurs taux de diffusion du substrat. Cependant, les étapes d'optimisation doivent être effectuées pour chaque nouveau système et des résultats comme la résistance mécanique de la membrane peuvent jouer un rôle dans le choix final des conditions optimales.

2) Optimisation des conditions de la mesure

A l'aide des membranes réalisées dans les conditions optimales déterminées ci-dessus (10 UI pour 35 cm² ; temps de réticulation : 3 minutes ; pourcentage de gélatine : 5 % ; et concentration de substrat de 0,5 mM), on a étudié :

a) L'effet de la molarité du tampon phosphate :

La figure 7 représente les courbes (1) à (3) donnant la réponse du biocapteur selon l'invention en fonction de la concentration du tampon phosphate (pH 7,1) pour trois membranes différentes utilisant des quantités variables d'enzyme dans les conditions communes suivantes :

. Temps de réticulation à l'aide d'une solution de glutaraldéhyde à 1,25 % (poids/volume) : 3 minutes ;
. Concentration de la solution de gélatine : 5 % (poids/volume) ;
. Température : 21°C ;
. Concentration en L-lactate : 0,5 mM.

Légende de la figure 7 :

. Abscisse : concentration du tampon phosphate (en mM) ;
. Ordonnée : pente Smax (définie sur la figure 3) ;
. Quantité d'enzyme (UI pour 35 cm²) pour les courbes (1) à (3) : respectivement 1 ; 4 ; et 10.

Il est ainsi montré que la molarité du tampon aux concentrations étudiées n'a que très peu d'effet sur la réponse de l'électrode.

Par ailleurs, quand on augmente la force ionique d'un tampon phosphate 0,1 M, pH 7,1 en ajoutant des quantités croissantes de NaCl (0,1 à 2,0M), la réponse de l'électrode ne varie pas (courbe non présentée). La durée de l'étape de rinçage de la membrane doit cependant être augmentée et ceci probablement en liaison avec une augmentation de la viscosité de la solution.

Il convient de noter que des variations de la force ionique ou du pH du tampon peuvent avoir lieu, par exemple, lors d'une mesure directe sur un milieu de concentration saline aussi variable que celle du stratum corneum. Les résultats ci-dessus montrent que de telles variations n'ont que très peu d'influence sur la réponse de l'électrode.

b) L'effet du pH du tampon de mesure sur la réponse de l'électrode.

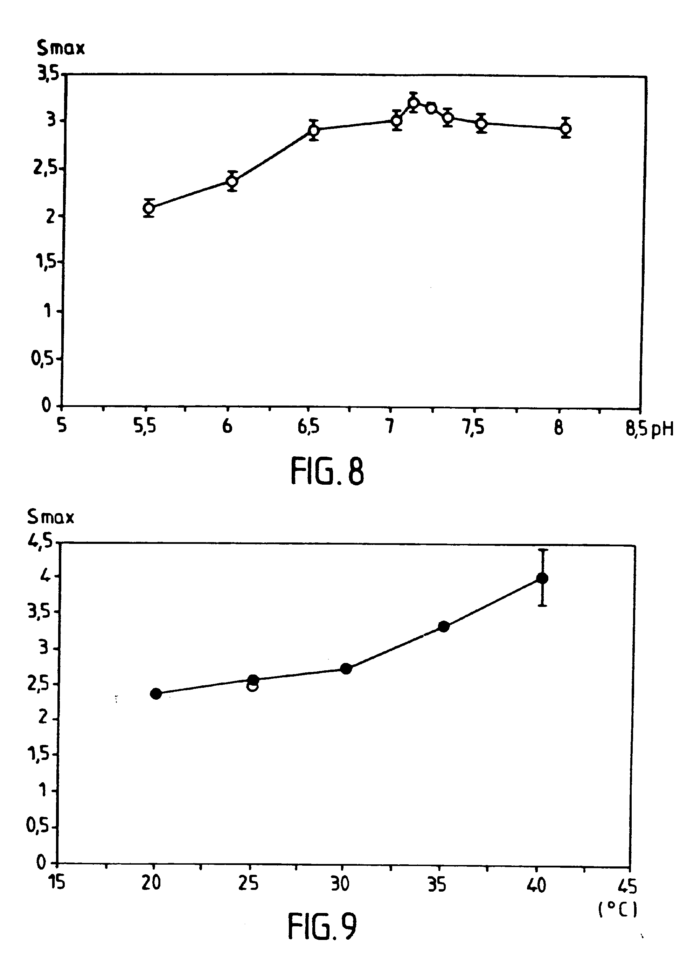

La figure 8 représente la réponse du biocapteur (valeur de la pente Smax définie sur la figure 3) en fonction du pH, chaque point représentant la moyenne de quatre déterminations, dans les conditions communes suivantes :

. Membrane à 10 UI pour 35 cm² ;
. Tampon phosphate : 0,1 M ;
. Temps de réticulation à l'aide d'une solution de glutaraldéhyde à 1,25 % (poids/volume) : 3 minutes ;
. Concentration de la solution de gélatine : 5 % (poids/volume) ;
. Température : 21°C ;

. Concentration en L-lactate : 0,5 mM.

Il est ainsi montré que, quand le pH varie entre 6,5 et 8, on ne note pas de changement important dans la réponse de l'électrode. La réponse est maximum pour un pH de 7,1 dans les conditions du test.

c) L'effet de la température du milieu de mesure :

La figure 9 représente la réponse du biocapteur (valeur de la pente Smax définie sur la figure 3) en fonction de la température, chaque point noir représentant la moyenne de quatre déterminations, dans les conditions communes suivantes :

. Membrane à 10 UI pour 35 cm$^2$ ;
. Tampon phosphate : 0,1M, pH 7,1 ;
. Temps de réticulation à l'aide d'une solution de glutaraldéhyde à 1,25 % (poids/volume) : 3 minutes ;
. Concentration de la solution de gélatine : 5 % (poids/volume) ;
. Concentration en L-lactate : 0,5mM.

Le témoin (point cerclé) constitue une vérification de la stabilité de l'enzyme, dans les conditions précitées, au bout d'un cycle de test de température allant jusqu'à 50°C, et avec un retour à 25°C. Une augmentation de température poussée jusqu'à 50°C (temps de maintien à cette température : environ 15 minutes) n'a pas causé de modification notable de la réponse de l'électrode ramenée à 25°C.

Comme le montre la figure 9, augmenter la température de 20°C à 40°C pour une concentration en lactate de 0,5 mM améliore la réponse de l'électrode. Cependant, pour des températures supérieures à 35°C, le pourcentage de variation de la mesure devient très important (supérieur à 10 % à 40°C) , ceci étant probablement lié au processus de dégazage de l'électrolyte. Toutes les expériences en flux continu ont donc été menées à 21°C. Ceci met en évidence l'importante stabilité thermique et mécanique de la membrane enzymatique du biocapteur selon l'invention.

## II. UTILISATION DU BIOCAPTEUR

### 1) Stabilité

a) Stabilité en conditions de stockage :

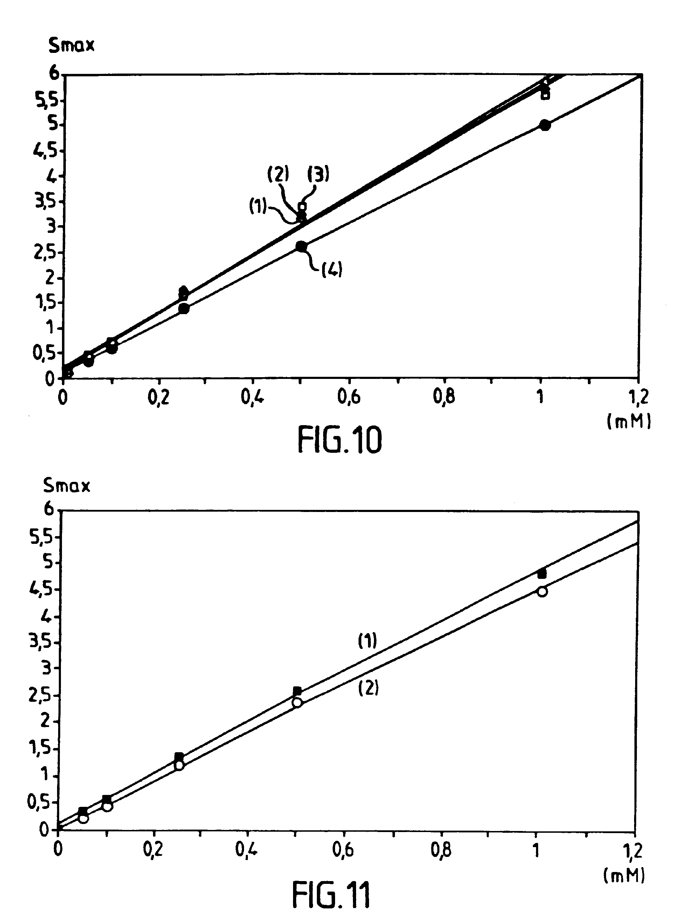

La figure 10 illustre la réponse en zone de linéarité, du biocapteur, à différents moments après la préparation de la membrane.

Légende de la figure 10

. Abscisse : concentration du L-lactate en mM ;
. Ordonnée : pente Smax définie sur la figure 3 ;
. Courbes (1) à (4) : respectivement 1 ; 9 ; 35 et 75 jours après la préparation de la membrane.

Les membranes enzymatiques sont stockées à 4°C dans le tampon de mesure sans azide de sodium ; aucune variation de la réponse de l'électrode n'est notée le premier mois. Jusqu'à 3 mois de stockage, la limite de détection et la zone de linéarité de la réponse sont conservées et des mesures peuvent toujours être effectuées de façon précise grâce à une courbe d'étalonnage appropriée.

b) Stabilité en opération continue :

La figure 11 illustre la réponse, en zone de linéarité du biocapteur après un fonctionnement de 15 jours.

Légende de la figure 11 :

. Abscisse : concentration de L-lactate en mM ;
. Ordonnée : pente Smax définie sur la figure 3 ;
  (1) : courbe d'étalonnage de référence
  (2) : au bout de 15 jours.

La stabilité de la réponse du biocapteur a été testée en cycle continu avec des concentrations standard de L-lactate de 2mM. Huit cent cycles de mesure (environ 1 minute chacun) ont pu être effectués sans modification notable de la réponse (2 séries d'expériences). Dans le système à flux continu, la stabilité opérationnelle exceptionnelle peut en partie être attribuée au fait que le temps de contact avec le L-lactate est réduit à 10 secondes par cycle. Les oxydases présentent souvent des cinétiques d'auto-inactivation, par perte de co-facteur (FAD), par l'$H_2O_2$ ou par les espèces actives de l'oxygène produites pendant la réaction enzymatique. L'auto-inactivation est d'autant plus sévère que la concentration des deux substrats est augmentée. Il est donc intéressant, pour obtenir des durées de stabilité maxima, de limiter autant que possible le temps de contact avec la solution de L-lactate.

c) Stabilité en opérations discontinues :

La stabilité du biocapteur a été testée pour des périodes alternées de stockage (4°C dans le tampon phosphate) et de mesures (mesures cutanées et en flux continu à 21°C). Même quand la sensibilité décroît de façon notable (après 15 jours et plus de 500 mesures), la limite de détection et la linéarité sont conservées et les mesures peuvent toujours être effectuées de façon précise.

Ces résultats font du biocapteur selon l'invention un système dont les variations de sensibilité peuvent être contrôlées par de simples étalonnages quotidiens, assurant ainsi la fiabilité des résultats obtenus.

2) Précision des mesures :

La variabilité des mesures effectuées en flux continu sous le contrôle d'un micro-ordinateur est inférieure à 4 % (reproductibilité parfaite des conditions pouvant influer sur la mesure ; pression d'injection de l'échantillon constante ; cycle parfaitement reproductible).

3) Spécificité :

On a comparé les résultats obtenus avec le biocapteur selon l'invention et ceux obtenus à partir de prélèvements soumis à une détermination du lactate par chromatographie en phase liquide. On a obtenu un coefficient de corrélation de 0,999 ce qui démontre le parfait fonctionnement du biocapteur selon l'invention et sa spécificité pour la mesure du L-lactate sans interférence des autres composés présents sur le revêtement cutané.

**Revendications**

1. Biocapteur à enzyme destiné à la mesure directe d'au moins un paramètre biochimique de la peau, ledit biocapteur comprenant, d'une part, une membrane dans laquelle est immobilisée une enzyme destinée à catalyser la transformation d'un substrat intervenant dans le métabolisme cutané, et, d'autre part, des moyens pour détecter et mesurer un phénomène provoqué par ladite transformation, qui est représentatif de la concentration du substrat à doser, celui-ci étant amené au contact de la membrane enzymatique (8), caractérisé par le fait qu'il comprend une cellule de mesure (13) comportant une ouverture, cellule dont une zone de paroi est constituée par ladite membrane enzymatique (8) et dont l'ouverture susmentionnée est apte à être obturée, lors de la mesure, par une zone de revêtement cutané (15), ladite cellule (13) étant associée à des moyens pour y faire circuler un liquide de mise en solution du substrat à doser.

2. Biocapteur selon la revendication 1, dans lequel la transformation du substrat à doser est une réaction qui consomme de l'oxygène et/ou produit de l'$H_2O_2$, caractérisé par le fait que les moyens de détection et de mesure sont constitués par une électrode (1) mesurant la diminution de la pression partielle d'oxygène ou l'augmentation de la concentration en $H_2O_2$, la membrane enzymatique (8) étant appliquée contre la partie sensible d'extrémité de ladite électrode (1), le liquide de mise en solution étant un tampon susceptible de fournir de l'oxygène.

3. Biocapteur selon la revendication 2, caractérisé par le fait que la cellule de mesure (13) est constituée par une bague (14), à une extrémité de laquelle est montée, de manière étanche, la partie sensible d'extrémité de ladite électrode (1), l'autre extrémité de ladite bague constituant l'ouverture de la cellule.

4. Biocapteur selon la revendication 2, destiné au dosage du L-lactate cutané, caractérisé par le fait que l'enzyme est la L-lactate oxydase, notamment la L-lactate oxydase de Pediococcus sp., et que l'électrode (1) est une électrode mesurant la diminution de la pression partielle d'oxygène.

5. Biocapteur selon l'une des revendications 1 à 4, caractérisé par le fait que la membrane enzymatique (8) est constituée d'un film-support (5) de matériau hydrophobe à perméabilité sélective vis-à-vis des gaz et d'un film porteur de l'enzyme formé par réticulation d'au moins une protéine inerte par un agent pontant, le film-support (5) de la membrane enzymatique (8) étant disposé du côté de l'électrode (1) alors que le film porteur de l'enzyme est disposé en vis-à-vis de l'ouverture de la cellule de mesure (13).

6. Biocapteur selon la revendication 5, caractérisé par le fait que la protéine inerte est la gélatine.

7. Biocapteur selon l'une des revendications 5 ou 6, caractérisé par le fait que l'agent pontant est le glutaraldéhyde.

8. Biocapteur selon les revendications 4 et 5 prises simultanément, seules ou en combinaison avec l'une des revendications 6 ou 7, caractérisé par le fait que le matériau hydrophobe constitutif du film-support (5) est le polypropylène ou le polytétrafluoréthylène.

9. Biocapteur selon les revendications 4 et 5 prises simultanément, seules ou en combinaison avec l'une des revendications 6 à 8, caractérisé par le fait que le film-support (5) a une épaisseur comprise entre 6 et 15 µm.

10. Biocapteur selon les revendications 4 et 5 prises simultanément, seules ou en combinaison avec l'une des revendications 6 à 9, caractérisé par le fait que le film porteur de l'enzyme a une épaisseur comprise entre 30 et 50 µm.

11. Biocapteur selon les revendications 4 et 5 prises simultanément, seules ou en combinaison avec l'une des revendications 6 à 10, caractérisé par le fait que le film porteur de l'enzyme contient de 0,15 à 0,50 UI de L-lactate oxydase par $cm^2$ de membrane.

12. Procédé de préparation d'une membrane enzymatique destinée à un biocapteur selon les revendications 4 à 7 et 10, prises simultanément, caractérisé par le fait que :
    a) on prépare une solution aqueuse renfermant, par ml, de 4 à 20 UI de L-lactate oxydase et de 30 à 70 mg de gélatine ;
    b) on étale ladite solution sur un film-support (5) à raison de 10 à 50 µl/$cm^2$ et on fait sécher ce revêtement ;
    c) sur le revêtement sec obtenu, on verse une solution aqueuse de glutaraldéhyde ayant une concentration comprise entre 0,5 et 2 % en poids et on laisse réticuler pendant un temps compris entre 1,5 et 4 minutes.

13. Procédé pour la mesure directe d'au moins un paramètre biochimique de la peau à l'aide d'un biocapteur selon l'une des revendications 1 à 11, caractérisé par le fait qu'on applique ledit biocapteur sur une zone choisie du revêtement cutané, de façon que l'ouverture de la cellule de mesure (13) soit fermée par ladite zone de revêtement cutané ; on injecte dans ladite cellule (13) un liquide apte à solubiliser le substrat cutané que l'on veut doser dont la composition favorise la catalyse ; après remplissage de la cellule de mesure (13), on effectue la détection et la mesure du phénomène provoqué par la transformation du substrat due à la catalyse de l'enzyme de la membrane (8) ; puis on fait circuler le liquide de solubilisation du substrat.

14. Procédé selon la revendication 13, caractérisé par le fait qu'on répète le cycle de mesure sur le même site, sans exposition de la membrane enzymatique (8) à l'air entre deux cycles successifs.

15. Procédé selon l'une des revendications 13 ou 14, utilisant un biocapteur selon la revendication 4, caractérisé par le fait que l'on utilise comme liquide de mise en solution du lactate cutané, un tampon ayant un pH compris entre 6 et 8 ; que l'on enregistre la variation de pression partielle d'oxygène $pO_2$ en fonction du temps pendant la période de non-circulation du tampon dans la cellule ; qu'on définit sur l'enregistrement le point A ayant pour abscisse le début d'injection du tampon et pour ordonnée la valeur maximum de $pO_2$ ; qu'on définit la droite D liant A au minimum B de la courbe enregistrée et qu'on mesure sa pente P pour l'utiliser comme valeur représentative du taux de lactate cutané.

16. Procédé selon la revendication 15, caractérisé par le fait que l'on arrête la circulation du tampon pendant un temps compris entre 3 et 6 minutes.

17. Utilisation du biocapteur selon l'une des revendications 1 à 11 pour l'estimation de l'hydratation cutanée.

**18.** Utilisation du biocapteur selon l'une des revendications 1 à 11 pour l'estimation de la sécrétion sudorale.

**Patentansprüche**

**1.** Enzym-Biosensor zur direkten Messung von wenigstens einem biochemischen Parameter der Haut, wobei der Biosensor einerseits eine Membran umfaßt, in der ein Enzym zur Katalyse der Umwandlung eines am Haut-Metabolismus beteiligten Substrates immobilisiert ist, und andererseits Mittel umfaßt zur Detektion und Messung eines durch diese Umwandlung bewirkten Ereignisses, das für die Konzentration des zu bestimmenden Substrates, das mit der Enzymmembran (8) in Kontakt gebracht wird, repräsentiv ist, dadurch gekennzeichnet, daß er eine Meßzelle (13), mit einer Öffnung umfaßt, wobei ein Wandabschnitt der Zelle von der Enzymmembran (8) gebildet wird und ihre oben erwähnte Öffnung bei der Messung durch einen Abschnitt der Oberhaut (15) abdichtbar ist, wobei die Zelle (13) mit Mitteln versehen ist, um eine Flüssigkeit zirkulieren zu lassen, die das zu bestimmende Substrat in Lösung bringt.

**2.** Biosensor nach Anspruch 1, worin die Umwandlung des zu bestimmenden Substrats eine Reaktion ist, die Sauerstoff verbraucht und/oder $H_2O_2$ produziert, dadurch gekennzeichnet, daß die Mittel zur Detektion und Messung von einer Elektrode (1) gebildet werden, die die Verminderung des Sauerstoff-Partialdruckes oder die Erhöhung der $H_2O_2$-Konzentration mißt, wobei die Enzymmembran (8) am sensiblen Endabschnitt der Elektrode (1) angebracht ist und das Lösungsmittel ein Puffer ist, der Sauerstoff zur Verfügung stellen kann.

**3.** Biosensor nach Anspruch 2, dadurch gekennzeichnet, daß die Meßzelle (13) von einer Buchse (14) gebildet wird, an deren einem Ende der sensible Endabschnitt der Elektrode (1) dicht angebracht ist und wobei das andere Ende der Buchse die Öffnung der Zelle bildet.

**4.** Biosensor nach Anspruch 2, zur Bestimmung von L-Lactat der Haut, dadurch gekennzeichnet, daß das Enzym L-Lactat-Oxidase ist, insbesondere L-Lactat-Oxidase von Pediococcus sp., und daß die Elektrode (1) eine Elektrode zur Messung der Verringerung des Sauerstoff-Partialdruckes ist.

**5.** Biosensor nach einem der Ansprüche 1 bis 4, dadurch gekennzeichnet, daß die Enzymmembran (8) aus einem Stützfilm (5) aus hydrophobem Material mit selektiver Gas-Permeabilität und aus einem Trägerfilm für das Enzym besteht, der durch Vernetzen wenigstens eines inerten Proteins mit einem Vernetzungsmittel gebildet wird, wobei der Stützfilm (5) der Enzymmembran (8) auf Seiten der Elektrode (1) angeordnet ist, wenn der Trägerfilm für das Enzym gegenüber der Öffnung der Meßzelle (13) angeordnet ist.

**6.** Biosensor nach Anspruch 5, dadurch gekennzeichnet, daß das inerte Protein Gelatine ist.

**7.** Biosensor nach einem der Ansprüche 5 oder 6, dadurch gekennzeichnet, daß das Vernetzungsmittel Glutaraldehyd ist.

**8.** Biosensor gemäß einer Kombination der Ansprüche 4 und 5, alleine oder in Kombination mit einem der Ansprüche 6 oder 7, dadurch gekennzeichnet, daß das den Stützfilm (5) bildende hydrophobe Material Polypropylen oder Polytetrafluorethylen ist.

**9.** Biosensor gemäß einer Kombination der Ansprüche 4 und 5, alleine oder in Kombination mit einem der Ansprüche 6 bis 8, dadurch gekennzeichnet, daß der Stützfilm (5) eine Dicke im Bereich von 6 bis 15 $\mu$m aufweist.

**10.** Biosensor gemäß einer Kombination der Ansprüche 4 und 5, alleine oder in Kombination mit einem der Ansprüche 6 bis 9, dadurch gekennzeichnet, daß der Trägerfilm für das Enzym eine Dicke im Bereich von 30 bis 50 $\mu$m aufweist.

**11.** Biosensor gemäß einer Kombination der Ansprüche 4 und 5, alleine oder in Kombination mit einem der Ansprüche 6 bis 10, dadurch gekennzeichnet, daß der Trägerfilm für das Enzym 0,15 bis 0,50 UI L-Lactat-Oxidase pro $cm^2$ Membran enthält.

**12.** Verfahren zur Herstellung einer Enzymmembran für einen Biosensor gemäß einer Kombination der Ansprüche 4 bis 7 und 10, dadurch gekennzeichnet, daß

a) man eine wäßrige Lösung herstellt, die pro ml 4 bis 20 UI L-Lactat-Oxidase und 30 bis 70 mg Gelatine enthält;

b) man diese Lösung auf einem Stützfilm (5) in einer Menge von 10 bis 50 $\mu$l/cm$^2$ aufträgt und man diesen Überzug trocknen läßt; und

c) man auf den erhaltenen trockenen Überzug eine wäßrige Glutaraldehydlösung mit einer Konzentration im Bereich von 0,5 bis 2 Gew.-% aufträgt und man eine Vernetzung über einen Zeitraum von 1,5 bis 4 Minuten durchführt.

13. Verfahren zur direkten Messung von mindestens einem biochemischen Parameter der Haut mit Hilfe eines Biosensors gemäß einem der Ansprüche 1 bis 11, dadurch gekennzeichnet, daß man den Biosensor auf einen ausgewählten Abschnitt der Oberhaut anwendet, so daß die Öffnung der Meßzelle (13) durch den Abschnitt der Oberhaut verschlossen wird; man in die Zelle (13) eine Flüssigkeit, welche zur Solubilisierung des Hautsubstrates, das man bestimmen will, geeignet ist und deren Zusammensetzung die Katalyse begünstigt, injiziert; man nach dem Auffüllen der Meßzelle (13) die Detektion und die Messung des Ereignisses durchführt, das durch die Umwandlung des Substrates aufgrund der Katalyse des Enzyms in der Membran (8) bewirkt wird; und man die Solubilisierungsflüssigkeit für das Substrat zirkulieren läßt.

14. Verfahren nach Anspruch 13, dadurch gekennzeichnet, daß man den Meßzyklus an der gleichen Stelle wiederholt, ohne die Enzymmembran (8) zwischen zwei aufeinanderfolgenden Zyklen der Luft auszusetzen.

15. Verfahren nach einem der Ansprüche 13 oder 14 unter Verwendung eines Biosensors gemäß Anspruch 4, dadurch gekennzeichnet, daß man als Flüssigkeit zum Lösen des Hautlactates einen Puffer mit einem pH im Bereich von 6 bis 8 verwendet; daß man die Veränderung des Sauerstoff-Partialdruckes $pO_2$ in Abhängigkeit von der Zeit während einer Phase registriert, in der der Puffer in der Zelle nicht zirkuliert; daß man für die Messung den Punkt A festlegt, der als Abszissenwert den Beginn der Pufferinjektion und als Ordinatenwert den $pO_2$-Maximalwert besitzt; daß man die Gerade D festlegt, welche A mit dem Minimum B der Meßkurve verbindet, und man deren Steigung P bestimmt, die man als repräsentativen Wert für den Hautlactatgehalt verwendet.

16. Verfahren nach Anspruch 15, dadurch gekennzeichnet, daß man die Pufferzirkulation über einen Zeitraum von 3 bis 6 Minuten stoppt.

17. Verwendung eines Biosensors nach einem der Ansprüche 1 bis 11 zur Bewertung der Hauthydratation.

18. Verwendung eines Biosensors nach einem der Ansprüche 1 bis 11 zur Bewertung der Schweißsekretion.

## Claims

1. Enzyme biosensor intended for the direct measurement of at least one biochemical parameter of the skin, the said biosensor comprising, on the one hand, a membrane in which is immobilised an enzyme intended for catalysing the conversion of a substrate participating in the metabolism of the skin, and, on the other hand, means for detecting and measuring a phenomenon caused by the said conversion, which phenomenon is representative of the concentration of the substrate to be assayed, the latter being brought into contact with the enzymatic membrane (8), characterised in that it comprises a measuring cell (13) with an opening, an area of the wall of the said cell being composed of the said enzymatic membrane (8) and the abovementioned opening thereof being capable of being blocked, during the measurement, by an area of skin covering (15), the said cell (13) being combined with means for circulating therein a liquid for dissolving the substrate to be assayed.

2. Biosensor according to Claim 1, in which the conversion of the substrate to be assayed is a reaction which consumes oxygen and/or produces $H_2O_2$, characterised in that the means of detection and measurement consist of an electrode (1) which measures the decrease in the partial pressure of oxygen or the increase in the concentration of $H_2O_2$, the enzymatic membrane (8) being applied against the sensitive part of the end of the said electrode (1), the dissolving liquid being a buffer which is capable of providing oxygen.

3. Biosensor according to Claim 2, characterised in that the measuring cell (13) consists of a ring (14) at one end of which is mounted, in a leakproof manner, the sensitive part of the end of the said electrode (1), the other end of the said ring forming the cell opening.

4. Biosensor according to Claim 2, intended for assaying skin L-lactate, characterised in that the enzyme is L-lactate oxidase, especially L-lactate oxidase from Pediococcus sp., and in that the electrode (1) is an electrode which measures the decrease in the partial pressure of oxygen.

5. Biosensor according to one of Claims 1 to 4, characterised in that the enzymatic membrane (8) consists of a supporting film (5) made of hydrophobic material which is selectively permeable to gases, and of an enzyme-bearing film formed by crosslinking at least one inert protein using a bridging agent, the supporting film (5) of the enzymatic membrane (8) being arranged on the side of the electrode (1) whereas the film bearing the enzyme is arranged opposite the opening of the measuring cell (13).

6. Biosensor according to Claim 5, characterised in that the inert protein is gelatin.

7. Biosensor according to one of Claims 5 or 6, characterised in that the bridging agent is glutaraldehyde.

8. Biosensor according to Claims 4 and 5, taken together, alone or in combination with one of Claims 6 or 7, characterised in that the constituent hydrophobic material of the supporting film (5) is polypropylene or polytetrafluoroethylene.

9. Biosensor according to Claims 4 and 5, taken together, alone or in combination with one of Claims 6 to 8, characterised in that the supporting film (5) has a thickness of between 6 and 15 $\mu$m.

10. Biosensor according to Claims 4 and 5, taken together, alone or in combination with one of Claims 6 to 9, characterised in that the film bearing the enzyme has a thickness of between 30 and 50 $\mu$m.

11. Biosensor according to Claims 4 and 5, taken together, alone or in combination with one of Claims 6 to 10, characterised in that the film bearing the enzyme contains from 0.15 to 0.50 IU of L-lactate oxidase per $cm^2$ of membrane.

12. Process for preparing an enzymatic membrane intended for a biosensor according to Claims 4 to 7 and 10, taken together, characterised in that:
   a) an aqueous solution is prepared containing, per ml, from 4 to 20 IU of L-lactate oxidase and from 30 to 70 mg of gelatin;
   b) the said solution is spread out on a supporting film (5) in an amount of from 10 to 50 $\mu$l/$cm^2$ and this coating is dried;
   c) an aqueous solution of glutaraldehyde having a concentration of between 0.5 and 2 % by weight is poured over the dry coating obtained and allowed to crosslink for a period of between 1.5 and 4 minutes.

13. Process for the direct measurement of at least one biochemical parameter of the skin using a biosensor according to one of Claims 1 to 11, characterised in that the said biosensor is applied to a selected area of the skin covering such that the opening of the measuring cell (13) is closed by the said area of skin covering; a liquid capable of solubilising the skin substrate which it is desired to assay, whose composition promotes catalysis, is injected into the said cell (13); after filling the measuring cell (13), the detection and the measurement of the phenomenon caused by conversion of the substrate due to catalysis by the enzyme of the membrane (8) are carried out; then the liquid for solubilising the substrate is circulated through.

14. Process according to Claim 13, characterised in that the measurement cycle is repeated at the same site without exposing the enzymatic membrane (8) to air between two successive cycles.

15. Process according to one of Claims 13 or 14, using a biosensor according to Claim 4, characterised in that a buffer having a pH of between 6 and 8 is used as liquid for dissolving the skin lactate; in that the variation in the partial pressure of oxygen $pO_2$ is recorded as a function of time during the period when the buffer is not circulated through the cell; in that the point A having as x-axis the start of buffer injection and as y-axis the maximum $pO_2$ value is defined on the recording; in that the straight line D Joining A to the minimum B of the recorded curve is defined and in that its slope P is measured so as to use it as the value representative of the level of skin lactate.

16. Process according to Claim 15, characterised in that the circulation of buffer is stopped for a period of between 3 and 6 minutes.

17. Use of the biosensor according to one of Claims 1 to 11 for estimating skin hydration.

18. Use of the biosensor according to one of Claims 1 to 11 for estimating sweat secretion.

FIG. 1

FIG. 1A

FIG. 2

FIG. 3

FIG. 4

FIG. 5

FIG. 6

FIG. 7

FIG. 8

FIG. 9

FIG.10

FIG.11

FIG. 12

FIG. 13